# EUROPEAN PATENT APPLICATION

(11) **EP 4 541 350 A2**
(43) Date of publication of application: **23.04.2025**
(21) Application number: 25162573.7
(22) Date of filing: 12.04.2021
(51) Int. Cl.: A61K 9/48

(54) **METHODS FOR OPTIMIZING TREATMENT OF MENTAL DISORDERS WITH CANNABIDIOL AND PHARMACEUTICAL COMPOSITIONS COMPRISING CANNABIDIOL**

(62) Divisional of application: 21167887.5
(71) Applicant: curantis UG, 51399 Burscheid (DE)
(72) Inventor: Leweke, Dr. Markus, Burscheid (DE)
(74) Representative: Richly & Ritschel Patentanwälte PartG mbB

(57) **Abstract**

Methods for optimizing treatment of mental disorders with cannabidiol and pharmaceutical compositions comprising cannabidiol are described.

## Description

### FIELD OF THE INVENTION

The present invention relates in vitro methods for optimizing treatment of mental disorders with cannabidiol and pharmaceutical compositions comprising cannabidiol.

### BACKGROUND INFORMATION

Mental disorders affect a large portion of the worldwide population. An example of a mental disorder is schizophrenia. About 1% of people worldwide suffer from schizophrenia. Because of the various forms of appearance, the diagnosis of schizophrenia is not easily made and diagnostic criteria were repeatedly revised. This results in differences for prevalence and incidence in the literature. Affected individuals suffer from positive and negative symptoms that include delusions, hallucinations, disturbances of affect and speech, illogical thinking, belief in supernatural powers as well as avolition, emotional blunting, poverty of speech, lack of motivation, poor body hygiene, anhedonia. Positive symptoms characterize an increase and negative symptoms a decrease of behavioral patterns compared to healthy persons. The symptom severity can be measured with, for example, the Positive and Negative Syndrome Scale (PANSS).

Moreover, schizophrenia is a disorder with a complex nature. Several neurotransmitters including dopamine, glutamate, serotonin, and endocannabinoids are involved. None of the approved antipsychotics can reduce all symptoms. Hence, the need for improved pharmacological therapies and with less side effects still exists.

### SUMMARY OF THE INVENTION

It was surprisingly found that a particular range of cannabidiol concentrations in the blood of the patients improves the symptoms of patients suffering from a mental disorder. Moreover, it was found that a particular preparation of CBD is especially useful in the treatment of subjects.

Therefore, the invention is directed to an in vitro method for determining efficacy of treatment of a subject having a mental disorder by administration of cannabidiol, comprising determining in vitro a level of cannabidiol in a sample from said subject having said mental disorder, wherein said treatment is considered efficient if the level of cannabidiol is at least about 25 µg/l.

The cannabidiol level can be at least about 30 µg/, at least about 35 µg/l, at least about 40 µg/l, at least about 50 µg/l, at least about 60 µg/l, at least about 70 µg/l, at least about 75 µg/l, at least about 80 µg/l, at least about 85 µg/l, or at least about 90 µg/l.

The cannabidiol level should usually not exceed above 1000 µg/l.

The cannabidiol level can be up to 1000 µg/l, 900 µg/l, 800 µg/l, 700 µg/l, 600 µg/l, 500 µg/l, or 400 µg/l.

The cannabidiol level can be determined about 3 to 5 weeks or about 4 weeks after the commencement of the treatment. In that case the cannabidiol level can be in the range of about 20 µg/l to about 400 µg/l, about 25 µg/l to about 360 µg/l, about 30 µg/l to about 200 µg/l, about 60 µg/l to about 120 µg/l, about 70 µg/l to about 110 µg/l, about 80 µg/l to about 100 µg/l, about 85 µg/l to about 95 µg/l, or about 90 µg/l.

The cannabidiol level can be determined about 1 to less than 3 weeks or about 2 weeks after the commencement of the treatment. In that case the cannabidiol level can be in the range of about 20 µg/l to about 60 µg/l, about 25 µg/l to about 55 µg/l, about 30 µg/l to about 50 µg/l, about 35 µg/l to about 50 µg/l, or about 45 µg/l.

The method can be useful for optimizing and/or monitoring efficacy of said treatment.

The mental disorder can be a psychotic disorder.

The psychotic disorder can be selected from the group consisting of schizophrenia, delusional disorder, or schizoaffective disorder, preferably schizophrenia.

The level of cannabidiol can be determined by liquid chromatography coupled to tandem mass spectrometry.

The level can be determined every 12-48 hours or determined every about 24 hours.

The level can be determined for the first time at least 5 days, 6, 7, 8, 9, 10, 11, 12, 13, 14 days after the commencement of a treatment with cannabidiol.

The cannabidiol can be administered to the subject at a dose of 5 mg to 1200 mg per day, 6 mg to 1200 mg per day, 7 mg/day to 1200 mg/day, or wherein said cannabidiol is to be administered to the subject at a dose of 8 to 12 mg per kg body weight per day.

A dose of cannabidiol which is to be administered within a predetermined time period can be increased if the determined level of cannabidiol is below the range of claim 1.

A dose of cannabidiol which is to be administered within a predetermined time period can be decreased if the determined level of cannabidiol is above the range of claim 1.

A dose of cannabidiol which is to be administered is to be administered intravenously, orally, sublingually, nasally, rectally, topically or by inhalation, or wherein a dose of cannabidiol which is to be administered is to be administered as a capsule.

The invention also relates to a pharmaceutical composition comprising at least 99% cannabidiol without any added excipients and having a particle size of less than 0.8 mm. The cannabidiol can be plant-derived or synthetic.

A capsule can comprise said pharmaceutical composition.

Said pharmaceutical composition or said capsule can be used in the treatment of a mental disorder, preferably schizophrenia.

### DETAILED DESCRIPTION OF THE INVENTION

Cannabidiol (CBD) is a highly lipophilic cannabinoid from the hemp plant Cannabis sativa. Compared to the well-known compound Δ⁹-THC it is non-psychotomimetic. The CBD to be used in the practice of the invention can be a plant derived, i.e. extracted from a plant source, or a synthetic CBD, i.e. chemically synthesized.

In the context of the invention CBD is preferentially chemically synthesized.

The invention is directed to an in vitro method for determining efficacy of treatment of a subject having a mental disorder by administration of cannabidiol, comprising determining in vitro a level of cannabidiol in a sample from said subject having said mental disorder, wherein said treatment is considered efficient if the level of cannabidiol is at least about 25 µg/l. This range maybe defined as the target range.

The sample can be a blood sample of the subject

The cannabidiol level (target) can be at least about 35 µg/l, at least about 30 µg/l, at least about 35 µg/l, at least about 40 µg/l, at least about 50 µg/l, at least about 60 µg/l, at least about 70 µg/l, at least about 75 µg/l, at least about 80 µg/l, at least about 85 µg/l, or at least about 90 µg/l.

The cannabidiol level should usually not exceed above 1000 µg/l.

The cannabidiol level can be up to 1000 µg/l, 900 µg/l, 800 µg/l, 700 µg/l, 600 µg/l, 500 µg/l, or 400 µg/l.

The cannabidiol level can be determined about 3 to 5 weeks or about 4 weeks after the commencement of the treatment. In that case the cannabidiol level can be in the range of about about 30 µg/l to about 360 µg/l, about 35 µg/l to about 200 µg/l, about 60 µg/l to about 120 µg/l, about 70 µg/l to about 110 µg/l, about 80 µg/l to about 100 µg/l, about 85 µg/l to about 95 µg/l, or about 90 µg/l.

The cannabidiol level can be determined about 1 to less than 3 weeks or about 2 weeks after the commencement of the treatment. In that case the cannabidiol level can be in the range of about 20 µg/l to about 60 µg/l, about 25 µg/l to about 55 µg/l, about 30 µg/l to about 50 µg/l, about 35 µg/l to about 50 µg/l, or about 45 µg/l.

The method can be useful for optimizing and/or monitoring efficacy of said treatment.

The mental disorder can be a psychotic disorder.

The psychotic disorder can be selected from the group consisting of schizophrenia, delusional disorder, or schizoaffective disorder.

The method can be preferably be used on samples obtained from subjects suffering from schizophrenia.

The level of cannabidiol can be determined by gas chromatography coupled to tandem mass spectrometry. However, any other method suitable to determine a level of cannabidiol in a sample of a subject can be used.

A sample can be obtained once after the administration of CBD or at defined, for example, regular intervals during treatment.

For example, the level can be determined every about 12-48 hours or determined every about 24 hours.

The level can be determined for the first time at least 5 days, 6, 7, 8, 9, 10, 11, 12, 13, 14 days after the commencement of a treatment with cannabidiol. Preferably the level is determined for the first time between day 7 and day 14, more preferably at about day 7 or about day 14, after the commencement of a treatment with cannabidiol.

Treatment can comprise administering to a subject a dose of 5 mg to 1200 mg per day. For example the dose can be 5 mg, 6 mg, 7 mg, 8 mg, 10 mg, 50 mg, 100 mg, 200 mg, 300 mg, 400 mg, 500 mg, 600 mg, 700 mg, 800 mg, 900, 1000, 1100 mg, or 1200 mg per day or any range of doses defined by the afore-mentioned doses. Preferably, the dose can be 600 to 1000 mg per day, for example, 800 mg to 1000 mg per day.

Alternatively or in addition, the cannabidiol can be administered to the subject at a dose of 8 to 12 mg per kg body weight per day, for example at 8, 9, 10, 11 or 12 mg per kg body weight per day.

The administration period can be at least 4 to 8 weeks, preferably at least 6 weeks.

CBD can be administered in any suitable pharmaceutical administration form, for example, as a tablet, capsule, gel, infusion or a combination thereof. Preferably, CBD can be administered as a capsule.

The pharmaceutical administration form can comprise about 5 to about 300 mg, 100 to about 300 mg, about 150 to about 250 mg, about 175 mg to about 225 mg, or about 200 mg CBD. Several administration forms may be combined to provide the required daily dose. For example a pharmaceutical administration form, for example, a capsule, comprising a predefined amount of CBD e.g. 200 mg, that is lower than the entire predetermined daily dose, e.g. 1000 mg, may be administered multiple times during a day, preferentially an equally spaced time intervals, e.g. 5 times per day.

A dose of cannabidiol which is to be administered within a predetermined time period can be increased if the determined level of cannabidiol is determined to be below the target range. For example, the dose daily dose can be increased, by or at least 10%, 20%, 30%, 40%, 50%, 60%, 70%, 80%, 90% or 100%, or by 10-100%.

A dose of cannabidiol which is to be administered within a predetermined time period can be decreased if the determined level of cannabidiol is above the target range. For example, the dose daily dose can be decreased, by or at least 1%, 2%, 3%, 5%, 10%, 20%, 30%, 40%, or 50, or by 1-50%.

The increase or decrease of the dose or daily dose can be dependent on the size of the respective dosage form. A dosage form is the smallest pharmaceutical unit that is administered to a subject, like a capsule, tablet, or the like. For example, if 1000 mg/ day are administered in five dosage forms of 200 mg per dose, a decrease or increase of one dose can be 200 mg, and thus 20%. Accordingly, the increase or decrease is dependent on the dosage form used.

A dose of cannabidiol which is to be administered is to be administered intravenously, orally, sublingually, nasally, rectally, topically or by inhalation, preferably orally. In particular, it is preferred that the dose of cannabidiol which is to be administered is to be administered as a capsule.

The invention also relates to a pharmaceutical composition which is particularly useful in performing the above-described method or methods.

Thus, the invention also relates to a pharmaceutical composition comprising cannabidiol without any added excipients and having a particle size of less than 0.8 mm. The composition can comprise at least about 99%, about 99% to about 99.9%, about 99.3% to 99.8% CBD. The cannabidiol can be a synthetic or a plant derived cannabidiol.

Without any added excipients means that no excipients, like, for example, lipids (e.g. triglycerides) or any form or liquids are added to the composition and thus comprised in the composition.

In a preferred embodiment, a capsule can comprise said pharmaceutical composition. The capsule is especially a capsule that prevents the transfer of liquids or lipids into the compartment formed by the capsule. A preferred material for the capsule is hydroxypropyl methylcellulose. The capsule can usually consist of two capsule pieces.

The pharmaceutical composition or said capsule can be used in the treatment of a mental disorder like those described above, preferably schizophrenia.

The pharmaceutical composition can be administered per day: 1, 2, 3, 4, 5, 6, 7, 8, 9, or 10 times according to the administration regimens described above.

Disclosed is also a method for producing the pharmaceutical composition comprising:
a. providing synthesized CBD;
b. transferring synthesized CBD into a first piece of the capsule of a two-piece capsule; and
c. contacting the first piece of the capsule with a second piece of the capsule to provide a complete two-piece capsule.

Preferably the step of transferring is achieved with a powder pipette. Preferably, the CBD is sieved after step a. and before step b. Preferably, the sieved used for sieving has a pore diameter of 0.8 mm, 0.7 mm, 0.5 mm or less. Thus, the sieved CBD can have a particle size of less than 0.8 mm, 0.7 mm, 0.5 mm or less.

### EXAMPLES

### EXAMPLE1: Production of CBD capsule

Synthetic CBD was produced by THC Pharm GmbH The Health Concept, Frankfurt am Main, Germany).

Purity of the obtained CBD was determined to be at least 99% (99, 35 %).

It was found that synthetic CBD is produced in the form of flakes or/and particles of having a very heterogeneous size distribution. This form did not allow to efficiently prepare pharmaceutical preparations like capsules and the like. Therefore, the synthetic CBD was subjected to a sieve process.

The particle size of the CBD can be reducing using any known device to reduce particle size of solids. For example, the CB can be reduced in size by a grinding device or a shearing device.

The CBD was sieved with a sieve of pore size of 0.77 mm. This pore size appears to be highly useful in the preparation of pharmaceutical preparations of CBD, especially capsules comprising the CPC preparations.

The sieved CBD was used without any additives in the production of capsules.

In particular, the sieved CBD was aspired with a powder pipette and then transferred into a first piece of a two-piece capsule using a powder pipette, the CBD content was expelled from the powder pipette. The capsule half was subsequently closed with a second piece of the two-piece capsule.

At none of the steps any further liquids (especially lipophilic liquids) were added.

The CBD dose per capsule was 200 mg.

The material of the capsule was hydroxypropyl methylcellulose (HPMC).

### EXAMPLE 2: Clinical trials and their evaluation

### Data

Data used for this research question are derived from the clinical trials, CBD-CT1 (ClinicalTrials.gov Identifier: NCT00628290) and CBD-PT (ClinicalTrials.gov Identifier: NCT00309413), which were all supervised by the same principal investigator (PI). Further information about the three included clinical trials is given in the following sections.

The Active Pharmaceutical Ingredient (API) grade CBD used to produce the study medication according to the Good Manufacturing Practice (GMP) guidelines was purchased from THC Pharm GmbH (Frankfurt am Main, Germany). For CBD-CT1 and CBD-PT the pharmacy of the University Hospital Cologne (Cologne, Germany) was the manufacturer of the study medication in line with the German Medicines Law (AMG).

### Study CBD-CT1

'Evaluation of the Antipsychotic Efficacy of Cannabidiol in Acute Schizophrenic Psychosis' is a double-blind, controlled, randomized, phase II trial with a parallel group design and was conducted from March 2001 to November 2004 (ClinicalTrials.gov Identifier: NCT00628290). The Ethics Committee of the Faculty of Medicine of University of Cologne approved the clinical trial with application number 00-074 on 08.06.2000.

A planned number of 42 patients with schizophrenic psychosis was randomized into two treatment arms and either treated with CBD (n = 21) or amisulpride (standard therapy, n = 21) for four weeks. All randomized patients complied with all inclusion and exclusion criteria: age between 18 and 50, male and female, persisting inpatient hospitalization, specific DSM-IV diagnoses, initial Brief Psychiatric Rating Scale (BPRS) score ≥ 36, understanding of the study objectives and the course of the study, legal capacity, no pregnancy or lactation phase, reliable contraception.

CBD was administered as an oral dose in a capsule formulation. The dose was increased during the first week from 200 mg/d to 800 mg/d (4 x 200 mg) and remained at this level for the rest of the study period. Due to serious unwanted effects, the dose could be reduced irreversibly to 600 mg/d starting with day 15. Collection of blood samples to quantify CBD trough plasma concentrations and completion of the PANSS were done at baseline, day 14 and day 28. Dose applications, blood sampling, and assessment of PANSS followed the time schedule presented below. Additional treatment with 7.5 mg lorazepam per day during the whole study period was permitted.

There are four out of n = 21 patients treated with CBD who are excluded from analysis: three patients have no measured CBD plasma concentrations at any time point and one patient has noticeable high CBD plasma concentrations on day 14 where validity is questionable. Another three patients dropped out after day 14 and no further CBD plasma concentrations and PANSS scores are available. Data from drop-out patients up to day 14 is included in the analysis. A total of 17 patients are included in the analysis.

The method used to measure the CBD plasma concentrations in this trial is described in Schreiber D. Auswirkungen von Cannabiskonsum auf das endogene Cannabinoidsystem: Entwicklung einer LC-MS/MS-Analytik zur Messung von Endocannabinoiden im Serum. Bonn: Rheinische Friedrich-Wilhelms-Universität Bonn, Bonn; 2007.

### Study CBD-PT

This is a phase II, placebo-controlled, randomized, cross-over clinical trial on the antipsychotic properties of cannabidiol (ClinicalTrials.gov Identifier: NCT00309413). On 28.07.2005 this clinical trial with application number 04-153 was approved by the Ethics Committee of the Faculty of Medicine of University of Cologne.

Eighteen patients with a diagnosis of schizophrenia or schizophreniform disorder should be included in this trial per protocol. Because dropouts had to be replaced by a new patient a total number of 29 patients was randomized from February 2006 to June 2008. All randomized patients complied with all inclusion and exclusion criteria: age between 18 and 65, male and female, specific DSM-IV diagnoses, initial BPRS score ≥ 36, no pregnancy or lactation phase, reliable contraception, no lack of accountability, no other relevant interferences, no treatment with depot-antipsychotics during the last three months, no severe internal or neurological illness, no QTc prolongation, no acute suicidality nor endangerment of others by the patient. Ten patients started with CBD treatment first and 19 patients with placebo. Treatment lasted four weeks: the first period with two weeks of CBD or placebo treatment was followed by a second period with a two-week treatment of placebo or CBD, respectively. There was no washout period between the two treatments. 600 mg/d (3 x 200 mg) of CBD were given as an oral dose in a capsule formulation. The dose could be reduced irreversibly to 400 mg/d in case of serious unwanted effects. Blood samples for quantification of CBD plasma concentration were taken at baseline and day 7, 14, 21, 28. The PANSS was applied at baseline and day 14 and 28. Dose applications, blood sampling, and assessment of PANSS followed the time schedule presented below. Additional treatment with 5.0 mg lorazepam per day during the whole study period was permitted.

A total of n = 11 patients dropped out during the study period. Seven patients dropped out in the first period under placebo treatment. They were never treated with CBD and therefore were excluded from the analysis of the present research project. One patient dropped out in the second period with CBD treatment but before any CBD plasma concentration was measured and therefore was excluded from analysis, too. Another three patients dropped out but have at least one or two CBD plasma concentrations measurements and can be included in the analysis. Altogether data from 21 patients are included in the analysis.

The method used to measure the CBD plasma concentrations in this trial is described in C. Schaefer: Exploration der Endocannabinoide und verwandter Fettsäureethanolamide im Serum von Patienten mit einer Borderline Persönlichkeitsstörung und/oder Posttraumatischen Belastungsstörung: Validierung einer LC-MS/MS-Methode. Cologne: University of Cologne; 2015.

### Data extraction and preparation

The clinical trials, CBD-CT1 and CBD-PT, focused on the antipsychotic effect of CBD and not on the PK characteristics. Therefore, documentation of time points when CBD was administered and when blood samples were taken was limited and not included in the electronic data. Information on possible dose reductions e.g. due to adverse events were missing in the electronic data, too. In cooperation with the PI, time points were inputted based on standard routines on the ward: application of study medication was set to 9:00 AM, 12:30 PM, 6:30 PM, and 10:00 PM, and blood sampling was scheduled at 8:45 AM. Assessment of the PANSS was also timed to 8:45 AM. The loss of accuracy by standardizing times was considered acceptable because the time interval between doses and blood sampling is long, so small deviations would have no major impact. Information on actually applied doses or reduced doses was updated in cooperation with a member of the PI's research group. Based on notes in archived patient's case report files and other paper-based documents the electronic data was adjusted.

For two patients in CBD-CT1 and CBD-PT the baseline CBD concentration was missing, and 20 patients had baseline values exceeding the local lower limit of quantification (0.12 µg/l). After consultation with the PI, these values were replaced with 0 because all patients with positive drug screening were excluded from the clinical trial according to the study protocol. Baseline values > 0 may result from errors in procedure e.g. when blood samples were taken after dose application. Therefore, the replacement is considered as being equivalent to an imputation of a predose measurement which was not conducted.

### Positive and Negative Syndrome Scale

The Positive and Negative Syndrome Scale (PANSS) is a psychopathological scale and was developed to assess the symptom severity in patients with schizophrenia. It was first described by Kay et al. in 1987 (Kay SR, Fiszbein A, Opler LA. "The positive and negative syndrome scale (PANSS) for schizophrenia". Schizophrenia bulletin. 1987;13(2):261-276.) and includes 30 questions which are a combination of 18 items of the BPRS and 12 items of the Psychopathology Rating Schedule (PSYRATS) (see Leucht S, Kane JM, Kissling W, Hamann J, Etschel E, Engel RR. "What does the PANSS mean?" Schizophrenia research. 2005;79(2-3):231-238.) All questions are rated on a 7-point scale from 1 = absence of symptoms to 7 = extremely distinct symptoms. Therefore, the PANSS total score (sum score of all 30 items) can range from 30 to 210. Here applies: the lower the PANSS score the better/healthier is the patient.

The original 3-factor solution suggested by Kay et al. for the PANSS which was used in CBD-CT1 and CBD-PT comprises a positive scale, a negative scale, and a general psychopathology scale. The positive scale contains delusion, disorganized thoughts, hallucination, excitement, grandiosity, suspiciousness/persecution, and animosity. Blunted affect, emotional withdrawal, poor rapport, passive/apathetic social withdrawal, difficulty in abstract thinking, stereotyped thinking, lack of spontaneity, and flow of conversation are part of the negative scale. The general psychopathology scale includes somatic concern, anxiety, guilt feelings, tension, mannerisms, abnormal posture, depression, motoric slowdown, uncooperativeness, unusual thought content, disorientation, poor attention, lack of judgment and insight, disturbance of willpower, poor impulse control, preoccupation and active social avoidance.

It takes about 50 minutes to complete the scale by a trained interviewer.

### Results

In clinical trials CBD-CT1 and CBD-PT, the psychopathological state of patients with schizophrenia was assessed using the PANSS at baseline and day 14 and 28.

Results of descriptive analysis of both total scores and the three PANSS subscores are given in Table 1. All mean scores decreased over time, with increasing standard deviation. The same can be observed for the change of scores from baseline (see Table 1): mean change and corresponding standard deviation both increase over time for all presented scores, thereby indicating an improvement of psychopathology at the group level.

Descriptive analysis of observed CBD plasma concentrations is presented in Table 2.

To verify this impression of improvement the PANSS was analyzed using a repeated measures analysis of variance. The normal distribution assumption at each point in time (baseline, day 14, day 28) was checked with a Shapiro-Wilk test (Sam S. Shapiro, Martin Bradbury Wilk: "An analysis of variance test for normality (for complete samples)", Biometrika, 52(3/4), 1965, pp. 591-61). For all four PANSS scores a significant effect of time within the individual was detected (p < 0.001) and thus confirms a statistically significant change in PANSS over time.

| **Table 1:** Descriptive analysis of PANSS score values per time point. Data are presented as mean ± SD. | | | | | |
|---|---|---|---|---|---|
| | N (CT1\|PT) | PANSS Total Score | PANSS Positive Score | PANSS Negative Score | PANSS General Score |
| Baseli ne | 38 (17\|21) | 90.4 ± 14.1 | 22.9 ± 4.3 | 22.5 ± 6.0 | 45.0 ± 8.2 |
| Day 14 | 36 (17\|19) | 76.9 ± 17.0 | 18.9 ± 4.5 | 19.0 ± 5.6 | 38.9 ± 9.6 |
| Day 28 | 24 (14\|10) | 68.9 ± 23.2 | 16.9 ± 5.4 | 16.1 ± 6.2 | 35.9 ± 13.1 |

| **Table 2:** Descriptive analysis of CBD plasma concentration per time point. Data are presented as count or mean ± SD, respectively. | | | | | |
|---|---|---|---|---|---|
| | N Total (CT1\|PT) | N under treatment Total (CT1\|PT) | Total CBD [µg/l] | CBD-CT1 CBD [µg/l] | CBD-PT CBD [µg/l] |
| Baseli ne | 38 (17\|21) | 38 (17\|21) | 0 - | 0 - | 0 - |
| Day 7 | 21 (0\|21) | 38 (17\|21) | 14.1 ± 9.1 | -- | 14.1 ± 9.1 |
| Day 14 | 33 (17\|16) | 36 (17\|19) | 35.4 ± 22.6 | 50.5 ± 21.2 | 19.3 ± 8.7 |
| Day 21 | 8 (0\|8) | 15 (15\|0) | 4.0 ± 1.2 | -- | 4.0 ± 1.2 |
| Day 28 | 21 (14\|7) | 15 (15\|0) | 29.2 ± 27.8 | 42.6 ± 24.7 | 2.3 ± 1.0 |

In addition, the correlation between the CBD plasma concentration and the change of the PANSS score at day 14 after commencement of CBD administration was estimated:

| **Table 3**: Estimated CBD plasma concentration in µg/l in relation to the percentage change of PANSS vis-á-vis the baseline score two weeks after commencement of CBD administration | | | | | | | | |
|---|---|---|---|---|---|---|---|---|
| | PANSS | | | | | | | |
| delta % | PANSS_T µg/l CBD average (range) | | PANSS_P µg/l CBD average (range) | | PANSS_N µg/l CBD average (range) | | PANSS_G µg/l CBD average (range) | |
| 10% | 17.5 | (13.4 ; 25.5) | 19.1 | (13.0 ; 35.7) | 17.8 | (12.9 ; 28.9) | 17.3 | (13.3 ; 24.6) |
| 20% | 35.0 | (26.7 ; 50.9) | 38.2 | (26.0 ; 71.4) | 35.6 | (25.7 ; 57.7) | 34.5 | (26.6 ; 49.2) |
| **25%** | **43.8** | **(33.4 ; 63.7)** | **47.7** | **(32.5 ; 89.3)** | **44.5** | **(32.2 ; 72.1)** | **43.1** | **(33.2 ; 61.5)** |
| 30% | 52.6 | (40.1 ; 76.4) | 57.2 | (39.1 ; 107.1) | 53.4 | (38.6 ; 86.6) | 51.8 | (39.8 ; 73.8) |
| 40% | 70.1 | (53.4 ; 101.9) | 76.3 | (52.1 ; 142.8) | 71.2 | (51.5 ; 115.4) | 69.0 | (53.1 ; 98.4) |
| *50%* | *87.6* | *(66.8 ; 127.3)* | *95.4* | *(65.1 ; 178.5)* | *89.0* | *(64.3 ; 144.3)* | *86.3* | *(66.4 ; 123.0)* |
| 60% | 105.1 | (80.1 ; 152.8) | 114.5 | (78.1 ; 214.2) | 106.8 | (77.2 ; 173.1) | 103.5 | (79.7 ; 147.6) |
| 70% | 122.6 | (93.5 ; 178.3) | 133.6 | (91.1 ; 249.9) | 124.6 | (90.1 ; 202.0) | 120.8 | (93.0 ; 172.2) |
| 75% | 131.4 | (100.1 ; 191.0) | 143.1 | (97.6 ; 267.8) | 133.5 | (96.5 ; 216.4) | 129.4 | (996 ; 184.5) |
| 80% | 140.2 | (106.8 ; 203.7) | 152.6 | (104.1 ; 285.6) | 142.4 | (102.9 ; 230.8) | 138.0 | (106.3 ; 196.8) |
| 90% | 157.7 | (120.2 ; 229.2) | 171.7 | (117.2 ; 321.3) | 160.2 | (115.8 ; 259.7) | 155.3 | (119.5 ; 221.4) |
| 100% | 175.2 | (133.5 ; 254.6) | 190.8 | (130.2 ; 357.0) | 178.0 | (128.7 ; 288.5) | 172.5 | (132.8 ; 246.0) |

In general, it is expected that a percentage change of 50% of the PANSS score(s) vis-á-vis the baseline score after four to six weeks of treatment indicates a significant improvement of the PANSS score(s) (see Correll C.U., "Quantifying clinical relevance in the treatment of schizophrenia, Clinical Therapeutics", Vol. 33, Number 12, 2011, B16-B39).

Since the PANSS score(s) cover symptoms experienced or observed during the preceding 7 days, the minimum required observation period is one week. To avoid an overlap to the observation of initial symptoms, a treatment period of 2 weeks is considered a minimum reliable period. Therefore, we analyzed the treatment effect after 2 weeks of treatment. Due to the analyzed shortened treatment period a percentage change of 25% of the PANSS score(s) vis-á-vis the baseline score indicates a significant improvement of the PANSS score(s). We found that a CBD plasma concentration of at least about 25 µg/l results in the required percentage change of the PANSS score(s).

## Claims

1. An in vitro method for determining efficacy of treatment of a subject having a mental disorder by administration of cannabidiol, comprising
determining in vitro a level of cannabidiol in a sample from said subject having said mental disorder,
wherein said treatment is considered efficient if the level of cannabidiol is at least 25 µg/l.

2. The method of claim 1, wherein said cannabidiol level is at least about 30 µg/, at least about 35 µg/l, at least about 40 µg/l, at least about 45 µg/l, at least about 50 µg/l, at least about 60 µg/l, at least about 70 µg/l, at least about 75 µg/l, at least about 80 µg/l, at least about 85 µg/l, or at least about 90 µg/l.

3. The method of claim 1 or 2, wherein said method is useful for optimizing and/or monitoring efficacy of said treatment.

4. The method of any of the previous claims, wherein said mental disorder is a psychotic disorder.

5. The method of claim 4, wherein said psychotic disorder is selected from the group consisting of schizophrenia, delusional disorder, or schizoaffective disorder, preferably schizophrenia.

6. The method of any of the previous claims, wherein said level of cannabidiol is determined by liquid chromatography coupled to tandem mass spectrometry.

7. The method of any of the previous claims, wherein said level is determined every 12-48 hours or determined every about 24 hours.

8. The method of any of the previous claims, wherein said level is determined for the first time at least 5 days, 6, 7, 8, 9, 10, 11, 12, 13, 14 days after the commencement of a treatment with cannabidiol.

9. The method of any of the previous claims, wherein said cannabidiol is to be administered to the subject at a dose of 5 mg to 1200 mg per day, or wherein said cannabidiol is to be administered to the subject at a dose of 8 to 12 mg per kg body weight per day.

10. The method of any of the previous claims, wherein a dose of cannabidiol which is to be administered within a predetermined time period is increased if the determined level of cannabidiol is below the range of claim 1.

11. The method of any of the previous claims, wherein a dose of cannabidiol which is to be administered within a predetermined time period is decreased if the determined level of cannabidiol is above the range of claim 1.

12. The method of any of the previous claims, wherein a dose of cannabidiol which is to be administered is to be administered intravenously, orally, sublingually, nasally, rectally, topically or by inhalation, or wherein a dose of cannabidiol which is to be administered is to be administered as a capsule.

13. A pharmaceutical composition comprising at least 99% cannabidiol without any added excipients having a particle size of less than 0.8 mm.

14. A capsule comprising the pharmaceutical composition of claim 13.

15. The pharmaceutical composition of claim 13 or the capsule of claim 14 for use in the treatment of a mental disorder, preferably schizophrenia.
